# EUROPÄISCHE PATENTANMELDUNG

(11) **EP 0 742 213 A1**
(43) Veröffentlichungstag der Anmeldung: **13.11.1996**
(21) Anmeldenummer: 96107059.6
(22) Anmeldetag: 06.05.1996
(51) Int. Cl.: C07D 239/96

(54) **Verfahren zur Herstellung von substituierten 3-Arylchinazolin-2,4-dionen**

(30) Priorität: 10.05.1995 DE 19517035; 10.05.1995 DE 19517036
(71) Anmelder: HOECHST AKTIENGESELLSCHAFT, 65929 Frankfurt am Main (DE)
(72) Erfinder: Weiguny, Jens, Dr., 64331 Weiterstadt (DE); Borchert, Holger, Dr., 65929 Frankfurt (DE); Gerdau, Thomas, Dr., 65817 Eppstein (DE)

(57) **Zusammenfassung**

Die Erfindung betrifft ein Verfahren zur Herstellung von substituierten 3-Arylchinazolin-2,4-dionen der allgemeinen Formel (I) 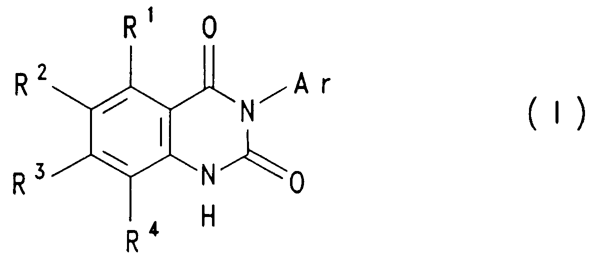 worin R¹, R², R³ und R⁴ wie in Anspruch 1 angegeben definiert sind und Ar für 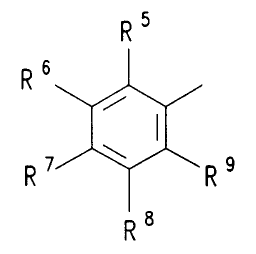 steht, wobei R⁵ bis R⁹ wie in Anspruch 1 angegeben definiert sind durch Umsetzung einer Verbindung der Formel (II) 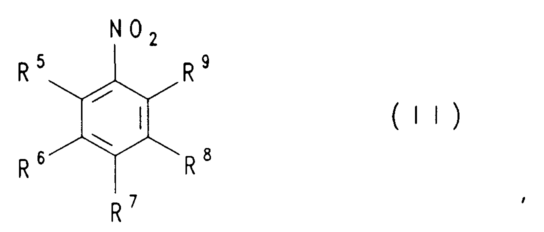 mit einer Verbindung Z in Gegenwart eines Katalysators, eines Liganden und eines Lösungsmittels, wobei Z steht für
a) ein Anthranilsäurederivat der Formel (III) 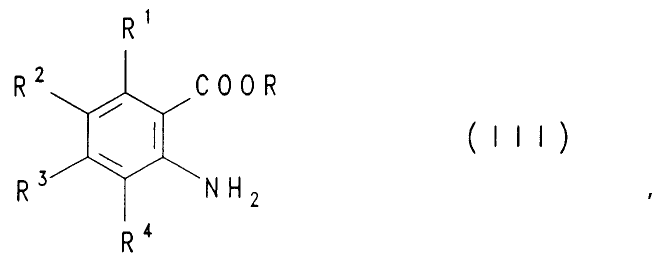 oder
b) einen Alkohol R¹⁰-OH und wobei im Falle b) anschließend das entstandene Carbamat mit einem Anthranilsäurederivat der Formel III umgesetzt wird.

## Beschreibung

Substituierte 3-Arylchinazolin-2,4-dione der allgemeinen Formel (I) sind interessante Zwischenprodukte für Pharmazeutika und Pflanzenschutzmittel (US 4.405.623; GB 1.059.271; EP 360.417).

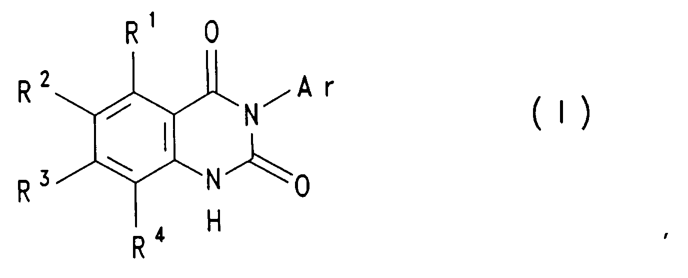

Die Herstellung von (I) erfolgt üblicherweise durch Reaktion von Anthranilsäure oder eines Anthranilsäurealkylester mit einem Arylisocyanat in einem gegenüber Isocyanaten inerten Reaktionsmedium.

Im Falle von Antrhanilsäuren erhält man so N-Arylcarbamoylanthranilsäuren, die zwischenisoliert, gegebenenfalls durch Umkristallisation gereinigt werden und dann in einem zweiten Reaktionsschritt, z. B in Polyphosphorsäure innerhalb von 5 Std. bei 150°C (EP 360 417) oder einem protischen, organischen Medium, wie z. B. Ethanol in Gegenwart überschüssiger, starker Mineralsäure, bevorzugt gasförmiger Salzsäure, zu 3-Arylchinazolin-2,4-dionen der Formel (I) ringgeschlossen werden (GB 1.059.271, Bsp. 3).
In entsprechender Weise liefern die Anthranilsäurealkylester bei der Umsetzung mit Arylisocyanaten N-Arylcarbamoylanthranilsäurealkylester, die zwischenisoliert werden und in analoger Weise wie die Säuren zu den 3-Arylchinazolin-2,4-dionen cyclisiert werden können. Es ist auch bekannt, die Cyclisierung zu (I), in protischen Medien wie z. B. Ethanol oder Methanol in Gegenwart von wäßrigem Natriumhydroxid durchzuführen (DOS 1.804.391, Bsp. 4; J. Heterocycl. Chem. 19(2), S. 269, 1982).

Die Arylisocyanate werden im allgemeinen durch Phosgenierung von den entsprechenden Anilinen dargestellt (Houben Weyl, Band E4, S. 741), welche wiederum aus den entsprechenden Nitroaromaten durch Reduktion gewonnen werden (Houben Weyl, Band 11/1, S. 360).

Der Einsatz von hochgiftigem Phosgen und damit der von Chlor, das nicht im Endprodukt erscheint, verteuert diesen zweistufigen Prozeß zusätzlich und bringt Probleme mit sich hinsichtlich des Umweltschutzes und der Sicherheit.

Insgesamt läuft die Synthese von (I) ausgehend von den entsprechenden Nitroaromaten über 4 Stufen und beinhaltet den Einsatz von giftigem Phosgen und Isocyanaten. Es bestand daher Bedarf, ein kürzeres, verbessertes Verfahren zur Herstellung von 3-Arylchinazolin-2,4-dionen zu entwickeln.

Diese Aufgabe wird gelöst durch ein Verfahren zur Herstellung von substituierten 3-Arylchinazolin-2,4 dionen der allgemeinen Formel (I) 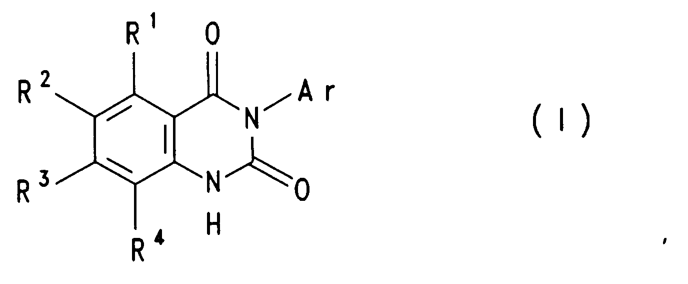 worin R¹, R², R³ und R⁴ unabhängig voneinander Wasserstoff, Halogen, (C₁-C₁₂)Alkyl, (C₁-C₁₂)Alkoxy, Ar oder ArO bedeuten und Ar für 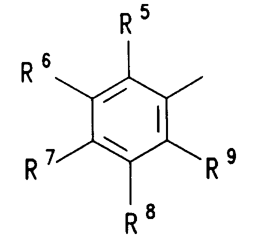 steht, wobei R⁵ bis R⁹ unabhängig voneinander Wasserstoff, Halogen, (C₁-C₁₂)Alkyl, (C₁-C₁₂)Alkoxy, Phenyl, Phenoxy bedeuten, wobei R⁵ und R⁶, R⁶ und R⁷, R⁷ und R⁸ oder R⁸ und R⁹ auch einen weiteren aromatischen Ring bilden können, dadurch gekennzeichnet, daß eine Verbindung der Formel (II) 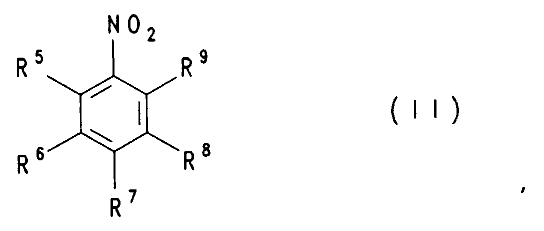 worin R⁵ bis R⁹ die oben erwähnte Bedeutung besitzen, mit Kohlenmonoxid und einer Verbindung Z in Gegenwart eines Katalystors, eines Liganden und eines Lösungsmittels umgesetzt wird, wobei Z steht für
a) ein Anthranilsäurederivat der Formel (III) 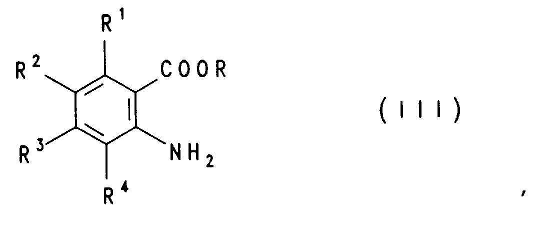 wobei R¹ bis R⁴ die oben erwähnte Bedeutung besitzen und R Wasserstoff oder (C₁-C₁₂)Alkyl bedeutet, oder
b) einen Alkohol der Formel (IV)

   R¹⁰-OH (IV)

   wobei R¹⁰ (C₁-C₁₂)Alkyl bedeutet und wobei im Falle b) anschließend das entstandene Carbamat der Formel (V) 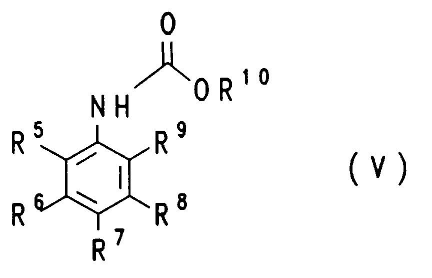 mit einem Anthranilsäurederivat der Formel (III) in Gegenwart einer Base und eines aprotischen Lösungsmittels umgesetzt wird.

Von Bedeutung ist das erfindungsgemäße Verfahren zur Herstellung von Verbindungen der Formel (I), worin R¹ bis R⁹ für Wasserstoff, Halogen, (C₁-C₆)Alkyl oder (C₁-C₆)Alkoxy steht, insbesondere zur Herstellung von 3-(2,4-Dichlorphenyl)-6-fluor-2,4-(1H,3H)-chinazolindion.

Die reduktive Carbonylierung wird in Gegenwart von Edelmetallen der 8. Nebengruppe, bevorzugt in Gegenwart von Pd (J. Organomet. Chem. 291, 117-27, 1985), Ru (J. Organomet. Chem. 451, 157-62, 1993) und Rh (J. Mol. Catal. 94, 195-206, 1994) und Liganden wie Pyridin, tert. Aminen oder Phosphinen und deren Derivaten, wobei zweizähnige Liganden, wie z. B. Bipyridin oder Phenanthrolinderivate, bevorzugt sind, durchgeführt. Als Phosphine können z. B. Bisdiphenylphosphinomethan, Bisdiphenylphosphinoethan, Bisdiphenylphosphinopropan, Bisdiphenylphosphinobutan oder Naphos eingesetzt werden. Die Edelmetalle können in Form ihrer Salze, wie z. B. der Halogenide, Acetate, Sulphate etc., ihrer Oxide oder in Form von Komplexen, wie z. B. den Carbonylkomplexen, vorliegen. Es ist auch möglich, den Katalysator in metallischer Form auf einem inerten Träger wie Aktivkohle oder Aluminiumoxid einzusetzen.

Es hat sich in vielen Fällen bewährt, einen Katalysator einzusetzen, der aus einer der erwähnten Metallverbindungen und einer Lewis- oder Brönstedtsäure besteht.
Als Säure können beispielsweise p-Toluolsulfonsäure, substituierte Benzoesäuren, wie z. B. 2,4,6-Trimethylbenzoesäure oder Pivalinsäure eingesetzt werden. Es hat sich als günstig erwiesen, 0,001 bis 10 mol%, insbesondere 0,01 bis 1 mol%, Katalysator bezogen auf Nitrogruppen in der Lösung einzusetzen. Für Ligand und Säure liegt die zweckmäßige Menge im Bereich von der 0,1 bis 100fachen Menge an eingesetztem Katalysator, muß aber nicht identisch sein.

Die Umsetzung der Verbindung der Formel (II) mit einer Verbindung Z wird bei Temperaturen von 75-250°C, insbesondere von 100-180°C, und CO-Drücken von 20 bis 500 bar, insbesondere 50 bis 200 bar, durchgeführt. Die Reaktion verläuft in aprotischen Reaktionsmedien wie z. B. aromatischen, aliphatischen oder cycloaliphatischen Kohlenwasserstoffen, die inerte Substituenten besitzen können wie z. B. Alkyl- oder Chlorsubstituenten, Heterocyclen oder Ketonen, insbesondere einkernigen Alkylaromaten, oder bei Normalbedingungen flüssigen Alkanen und Cycloalkanen. In vielen Fällen haben sich Toluol, Xylol, Dichlorbenzol in reiner Form oder als Isomerengemisch bewährt.

Für den Fall, daß Z für einen Alkohol der Formel (IV) steht, ist es allerdings zweckmäßig, diesem Alkohol auch als Lösungsmittel zu verwenden. Er kann dabei auch durch inerte Lösungsmittel wie z.B. Xylol, Toluol oder Dichlorbenzol verdünnt werden.

Für den Fall der Umsetzung einer Verbindung der Formel II mit einem Anthranilsäurederivat der Formel III, können beide Reaktionspartner zusammen vorgelegt werden, es ist jedoch auch möglich, das Anthranilsäurederivat während der Reaktion zuzudosieren. Es hat sich als zweckmäßig erwiesen, die Verbindungen (II) und (III) im Molverhältnis von 0,9 : 1 bis 1,1 : 1 einzusetzen.

Für den Fall, daß eine Verbindung der Formel II mit einem Alkohol der Formel IV umgesetzt wird werden die erhaltenen Carbamate der Formel (V) anschließend mit den Anthranilsäurederivaten der Formel (III) in Gegenwart einer Base und eines aprotischen Lösungsmittels cyclisiert. Hierbei hat es sich bewährt, die Verbindungen (V) und (III) im Molverhältnis von 0,9 : 1 bis 1,1 : 1 einzusetzen. Es hat sich als günstig erwiesen, als Base Alkali- oder Erdalkalimetallalkoholate, -amide, -hydride oder Tetraalkylammoniumhydroxide zu verwenden.
Als aprotisches Lösungsmittel kann z. B. ein aromatischer, aliphatischer oder cycloaliphatischer Kohlenwasserstoff, der auch inerte Substituenten wie z. B. Alkyl- oder Chlorsubstituenten tragen kann, eingesetzt. Gute Resultate liefern auch Heterocyclen oder Ketone, sofern sie gegenüber der Base inert sind, als Lösungsmittel. In vielen Fällen haben sich Toluol, Xylol oder Dichlorbenzol in reiner Form oder als Isomerengemisch bewährt.

Die als Base verwendeten Alkali- oder Erdalkalimetalle können in Substanz oder als Lösung in den entsprechenden Alkoholen eingesetzt werden. In vielen Fällen hat sich der Einsatz von Natriummethylat in Methanol bewährt. Verwendet man Alkali- oder Erdalkalimetallhydride oder -amide, so könen diese ebenfalls in Substanz oder als Suspension in einem inerten Solvent eingesetzt werden. Die verwendeten Basen werden üblicherweise in Mengen von 0.5 bis 95 mol% bezogen auf die Edukte eingesetzt. Es hat sich als günstig erwiesen, Mengen von 1 bis 20 mol%, insbesondere 5 bis 10 mol%, zu verwenden.
Die Reaktionstemperatur der Cyclisierung wird zweckmäßig so gewählt, daß der freiwerdende Alkohol aus dem Reaktionsmedium abdestilliert. Bevorzugt liegen die Temperaturen zwischen 100 und 200°C.

Der glatte Verlauf und die hohe Ausbeute des Cyclisierungsschrittes waren besonders überraschend, da bei der Umsetzung von N-Phenylalkylcarbamaten mit Anthranilsäure (Organic Preparations and Procedures Int. 10(1), 13-16, 1978) eine nur mäßige Ausbeute von bestenfalls 60 % beschrieben ist, wenn man Anthranilsäure mit N-Phenylalkylcarbamaten auf 180°C erhitzt. Zudem mußte das Carbamat im Überschuß zugegeben werden.

### Beispiel 1:

a) Reduktive Carbonylierung von 2,4-Dichlornitrobenzol:
   22.4 Gew. % 2,4-Dichlornitrobenzol, 2.0 Gew.-% Pd-C (5 %), 1.3 Gew.-% 2,4,6-Trimethylbenzoesäure und 0.4 Gew.-% 3,4,7,8-Tetramethyl-1,10-phenanthrolin werden in 73.8 Gew.-% Methanol gelöst und in einem HC-4 Autoclaven gefüllt. Es wird ein Druck von 100 bar CO eingestellt und die Temperatur auf 180°C erhöht. Nach 2 Stunden wird der Versuch abgebrochen und die Lösung gaschromatographisch untersucht. Der Umsatz betrug 100 % bei einer Selektivität zum N-2,4-Dichlorphenylmethylcarbamat von 85 %.
b) Herstellung von 3-(2,4-Dichlorphenyl)-6-fluor-2,4(1H,3H)-chinazolindion:
   8.5 Gew.-% N-2,4-Dichlorphenylcarbamat, 6.5 Gew.-% 5-Fluoranthranilsäuremethylester, 0.1 Gew.-% Natriummethylat werden in 84.9 Gew.-% o-Dichlorbenzol gelöst und auf 170°C erhitzt. Das entstehende Methanol wird dabei abdestilliert. Nach 6 Std. Reaktionszeit läßt man abkühlen. Den dabei entstehenden Niederschlag trennt man ab und erhält so 91.3 % Ausbeute an 3-(2,4-Dichlorphenyl)-6-fluor-2,4(1H,3H)chinazolindion.

### Beispiel 2:

Herstellung von 3-(2,4-Dichlorphenyl)-6-fluor-2,4(1H,3H)-chinazolindion:
17,2 Gew.-% 2,4-Dichlornitrobenzol, 15,6 Gew.-% 5-Fluoranthranilsäuremethylester, 1,5 Gew.-% Pd-C (5 %), 1,0 Gew.-% 2,4,6-Trimethylbenzoesäure und 0,4 Gew.-% 3,4,7,8-Tetramethyl-1,10-phenanthrolin werden in 64,3 Gew.-% Toluol gelöst und in einem HC-4 Autoclaven gefüllt. Es wird ein Druck von 100 bar CO eingestellt und die Temperatur auf 180°C erhöht. Nach 2 Stunden läßt man abkühlen, filtriert das Produkt mit der Aktivkohle zusammen ab und kristallisiert aus Dichlorbenzol um. Man erhält 51 % 3-(2,4-Dichlorphenyl)-6-fluor-2,4(1H,3H)-chinazolindion.

## Patentansprüche

1. Verfahren zur Herstellung von substituierten 3-Arylchinazolin-2,4 dionen der allgemeinen Formel (I) 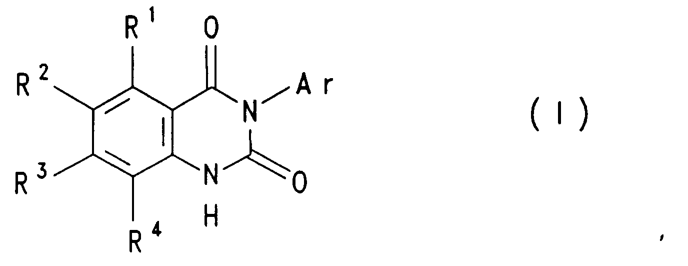 worin R¹, R², R³ und R⁴ unabhängig voneinander Wasserstoff, Halogen, (C₁-C₁₂)Alkyl, (C₁-C₁₂)Alkoxy, Ar oder ArO bedeuten und Ar für 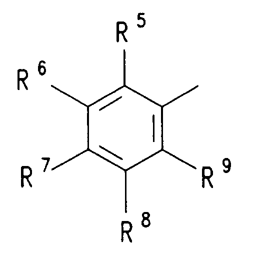 steht, wobei R⁵ bis R⁹ unabhängig voneinander Wasserstoff, Halogen, (C₁-C₁₂)Alkyl, (C₁-C₁₂)Alkoxy, Phenyl, Phenoxy bedeuten, wobei R⁵ und R⁶, R⁶ und R⁷, R⁷ und R⁸ oder R⁸ und R⁹ auch einen weiteren aromatischen Ring bilden können, dadurch gekennzeichnet, daß eine Verbindung der Formel (II) 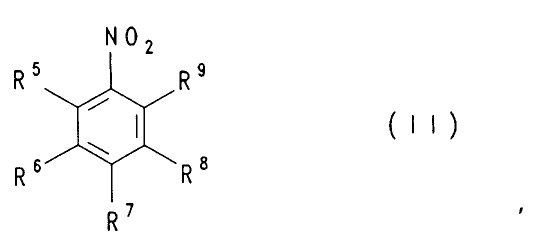 worin R⁵ bis R⁹ die oben erwähnte Bedeutung besitzen mit Kohlenmonoxid und einer Verbindung Z in Gegenwart eines Katalysators, eines Liganden und eines Lösungsmittels umgesetzt wird, wobei Z steht für
a) ein Anthranilsäurederivat der Formel (III) 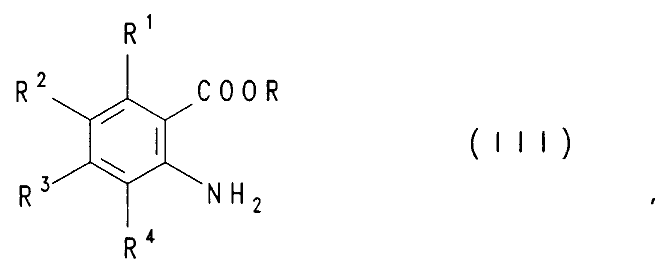 wobei R¹ bis R⁴ die oben erwähnte Bedeutung besitzen und R Wasserstoff oder (C₁-C₁₂)Alkyl bedeutet, oder
b) einen Alkohol der Formel (IV)
R¹⁰-OH (IV)
wobei R¹⁰ (C₁-C₁₂)Alkyl bedeutet und wobei im Falle b) anschließend das entstandene Carbamat der Formel (V) 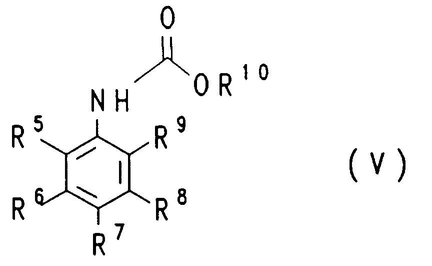 mit einem Anthranilsäurederivat der Formel (III) in Gegenwart einer Base und eines aprotischen Lösungsmittels umgesetzt wird.

2. Verfahren nach Anspruch 1, dadurch gekennzeichnet, daß R¹ bis R⁹ für Wasserstoff, Halogen, (C₁-C₆)Alkyl, (C₁-C₆)Alkoxy steht.

3. Verfahren nach Anspruch 1, dadurch gekennzeichnet, daß Formel (1) für 3-(2,4-Dichlorphenyl)-6-fluor-2,4(1H,3H)-chinazolindion steht.

4. Verfahren nach mindestens einem der Ansprüche 1 bis 3, dadurch gekennzeichnet, daß der Katalysator in eine Verbindung der Edelmetalle der 8. Nebengruppe, insbesondere Pd, Ru, Rh eingesetzt wird.

5. Verfahren nach mindestens einem der Ansprüche 1 bis 3, dadurch gekennzeichnet, daß als Katalysator ein Edelmetall der 8. Nebengruppe in metallischer Form auf einem inerten Träger eingesetzt wird.

6. Verfahren nach mindestens einem der Ansprüche 1 bis 5, dadurch gekennzeichnet, daß der Katalysator in einer Menge von 0,001 bis 10 Mol-%, insbesondere 0,01 bis 1 Mol-%, bezogen auf die Nitroverbindung eingesetzt wird.

7. Verfahren nach mindestens einem der Ansprüche 1 bis 6, dadurch gekennzeichnet, daß als Ligand ein Amin oder Phosphin eingesetzt wird.

8. Verfahren nach mindestens einem der Ansprüche 1 bis 7, dadurch gekennzeichnet, daß als Lösungsmittel aromatische Kohlenwasserstoffe, aliphatische Kohlenwasserstoffe, die jeweils auch Chlorsubstituenten tragen können, Heterocyclen oder Ketone, insbesondere Toluol, Xylol, Dichlorbenzol, eingesetzt werden, wobei für den Fall, daß Z für einen Alkohol der Formel (IV) steht, dieser auch als Lösungsmittel verwendet wird.

9. Verfahren nach mindestens einem der Ansprüche 1 bis 8, dadurch gekennzeichnet, daß bei der Umsetzung eines Carbamats der Formel (V) mit einem Anthranilsäurederivat der Formel (III) als Base Alkali- oder Erdalkalimetallalkoholate, -amide, -hydroxide oder Tetraalkylammoniumhydroxide, insbesonder Natriummethylat, eingesetzt werden.

10. Verfahren nach mindestens einem der Ansprüche 1 bis 8, dadurch gekennzeichnet, daß bei der Umsetzung eines Carbamats der Formel (V) mit einem Anthranilsäurederivat der Formel (III) als aprotisches Lösungsmittel aliphatische und aromatische Kohlenwasserstoffe, die Chlorsubstituenten besitzen können, Heterocyclen oder Ketone, insbesondere Toluol, Xylol oder Dichlorbenzol eingesetzt wird.
